# EUROPÄISCHE PATENTSCHRIFT

(11) **EP 1 795 217 B1**
(45) Veröffentlichungstag und Bekanntmachung des Hinweises auf die Patenterteilung: **17.02.2010**
(21) Anmeldenummer: 06121908.5
(22) Anmeldetag: 06.10.2006
(51) Int. Cl.: A61M 1/00

(54) **Absaugkanüle**
Suction cannula
Canule d'aspiration

(30) Priorität: 10.12.2005 DE 102005059088
(43) Veröffentlichungstag der Anmeldung: 13.06.2007
(73) Patentinhaber: Taufig, Ahmmed Ziah, 50668 Köln (DE)
(72) Erfinder: Taufig, Ahmmed Ziah, 50668 Köln (DE)
(74) Vertreter: Patentanwälte ter Smitten

(56) Entgegenhaltungen:
- DE-A1- 10 050 742
- DE-A1- 10 145 832
- DE-U1- 29 613 629

## Beschreibung

Die Erfindung bezieht sich auf eine Absaugkanüle mit einem Absaugrohr und mindestens einer Absaugöffnung zum Absaugen biologischen Gewebes aus einem lebenden menschlichen Körper.

Absaugkanülen dienen beispielsweise in Form von Absaugkanülen der Absaugung von Körpergewebe bzw. Körperfett, und weisen hierzu ein Absaugrohr und mindestens eine Absaugöffnung am distalen Ende des Absaugrohres auf, durch die das Körperfett aus dem Körperinneren abgesaugt werden kann. Die üblicherweise zur Liposuktion verwendeten Kanülen sind aus Edelstahl gefertigte mehrfach verwendbare Kanülen. Nach einer Liposuktion muss die Kanüle für die folgende Behandlung gereinigt und sterilisiert werden. Hierzu müssen das Innere und das Äußere der Kanüle zunächst mechanisch gereinigt werden. Das geschlossene Kanüleninnere kann nur durch Einleitung einer Spülflüssigkeit mechanisch gereinigt werden. Eine echte mechanische Reinigung des Kanülen-Inneren ist ohne relativ hohen Aufwand nicht möglich. Eine Sichtkontrolle des Reinigungserfolges bleibt in jedem Fall ausgeschlossen.

Bei der anschließenden Dampf-Sterilisation besteht die Gefahr, dass das Kanülen-Innere wegen seiner schlechten Zugänglichkeit nicht hinreichend sterilisiert wird.

Die Mehrzahl von Komplikationen bei der Liposuktion ist auf Infektionen zurückzuführen. Hiervor werden zu einem großen Anteil nicht-sterile Absaugkanülen verantwortlich gemacht.

DE 101 45 832 A1 beschreibt eine medizinisch-chirurgische Absaugkanüle für die Liposuktion. Die Absaugkanüle besteht aus einem starren Rohr mit seitlich angebrachten Absaugöffnungen. Die Kanülenspitze ist ein separates, in sich geschlossenes Bauteil, das mit der Kanülenröhre sicher, aber lösbar verbunden ist.

Aufgabe der Erfindung ist es, eine besser sterilisierbare Absaugkanüle zu schaffen.

Diese Aufgabe wird erfindungsgemäß von einer Absaugkanüle mit den Merkmalen des Patentanspruches 1 gelöst.

Bei der erfindungsgemäßen Absaugkanüle weist das Absaugrohr mindestens eine Reinigungsöffnung und einen Reinigungsöffnungs-Verschluss auf, der in seinem Schließzustand die Reinigungsöffnung verschließt und in seinem Öffnungszustand die Reinigungsöffnung freigibt. Die Absaugkanüle weist also nicht nur eine oder mehrere Absaugöffnungen vorzugsweise am distalen Ende auf, sondern weist ferner eine oder mehrere Reinigungsöffnungen zwischen dem distalen Ende des Absaugrohres und dem proximalen Ende des Absaugrohres, d. h. dem Ende, das an ein Absauggerät angeschlossen ist, auf. Durch die Reinigungsöffnung wird der Zugang zum Absaugrohr-Inneren verbessert. Die Reinigungsöffnung kann in einem Bereich des Absaugrohres vorgesehen werden, der erfahrungsgemäß besonders schlecht gereinigt oder dampf-sterilisiert wird. Dieser Bereich kann beispielsweise auf ungefähr halber Länge des Absaugrohres liegen. Der Sterilisationsdampf kann nun auch durch die Reinigungsöffnung eintreten, so dass das Absaugrohr auch in seinem mittleren Bereich gut sterilisierbar ist.

Die Reinigungsöffnung kann von der Öffnungs-Form und vom ÖffnungsQuerschnitt her an ein mechanisches Reinigungsinstrument angepasst sein, so dass hierdurch auch die mechanische Reinigung des Absaugrohr-Inneren erleichtert ist.

Mit dem Reinigungsöffnungs-Verschluss kann die Reinigungsöffnung vor der erneuten Benutzung der Kanüle zur Fettabsaugung wieder verschlossen werden, so dass durch die Reinigungsöffnung während einer Liposuktion keine Suktion erfolgt.

Vorzugsweise wird der Reinigungsöffnungs-Verschluss von einem Verschlussrohr gebildet, das in seinem Schließzustand außen auf dem Absaugrohr sitzt und die Reinigungsöffnung abdeckt. Im Öffnungszustand kann das Verschlussrohr axial von den Absaugrohr abgezogen sein, oder aber in Umfangsrichtung oder in Längsrichtung gegenüber dem Absaugrohr verdreht bzw. verschoben sein, um auf diese Weise die Reinigungsöffnung bzw. Reinigungsöffnungen durch Fluchtung mit entsprechenden Öffnungen des Verschluss-Rohres freizugeben.

Gemäß einer bevorzugten Ausgestaltung ist das Verschlussrohr ein Einmalartikel, der nach einmaligem Gebrauch nicht wieder verwendet, sondern entsorgt wird. Hierdurch entfällt die Sterilisation des Verschlussrohres für die folgende Anwendung. Durch die Ausbildung des Verschlussrohres als Einmalartikel kann das Verschlussrohr ausschließlich im Hinblick auf seine eigentlichen Funktionen konstruiert werden, nämlich dem einfachen Verschließen der Reinigungsöffnung, einer einfachen Handhabung und gegebenenfalls weiterer Funktionen. Das als Einmalartikel ausgebildete Verschlussrohr muss nicht im Hinblick auf seine mehrfache Sterilisierbarkeit konstruiert sein, insbesondere nicht im Hinblick darauf, auch von innen einfach mechanisch gereinigt werden zu können. Das Einmalartikel-Verschlussrohr weist daher keine separate Reinigungsöffnung auf. Im Gegensatz zu dem Verschlussrohr ist das in der Regel viel komplexere Absaugrohr mehrfach verwendbar.

Bevorzugt besteht das Absaugrohr aus Stahl und besteht das Verschlussrohr aus Kunststoff. Das Kunststoff-Absaugrohr ist preiswert im Spritzguss herstellbar, kann aber dennoch komplexe Strukturen aufweisen, ist für den einmaligen Gebrauch einfach sterilisierbar und kann günstige mechanische Eigenschaften aufweisen, insbesondere eine gewisse elastische Verformbarkeit, die die Montage und Fixierung des Verschlussrohres an dem Absaugrohr vereinfacht oder ermöglicht.

Die Befestigung des Einmal-Absaugrohres kann beispielsweise durch ein Innengewinde an dem Verschlussrohr erfolgen, das auf ein entsprechendes Außengewinde des Absaugrohres aufgeschraubt wird. Alternativ kann das Verschlussrohr innenseitig Ringnuten aufweisen, die mit entsprechenden außenseitigen Ringstegen des Absaugrohres verrastend zusammenwirken, um das Verschlussrohr in axialer und ggf. in Umfangs-Richtung zu fixieren.

Das Verschlussrohr kann auch durch entsprechende Klemm- oder Klapphebel oder eine Überwurfmutter an dem Absaugrohr fixiert werden.

Gemäß einer bevorzugten Ausgestaltung wird die Reinigungsöffnung von einem Längsschlitz in dem Absaugrohr gebildet. Es kann ein einziger Längsschnitt über nahezu die gesamte Länge des Absaugrohres vorgesehen sein. Um die Stabilität des Absaugrohres zu erhalten, können auch mehrere Längsschlitze axial hintereinander oder aber sich axial überdeckend und radial versetzt zueinander angeordnet sein. Der Längsschlitz kann sich bis zum distalen und proximalen Ende des Absaugrohres erstrecken.

Vorzugsweise weist auch das Verschlussrohr mindestens eine Reinigungsöffnung auf. Das Verschlussrohr kann zwar als Einmalartikel ausgebildet sein, das nach einer Liposuktion entfernt und weggeworfen und nach der Sterilisation des Absaugrohres in Form eines neuen sterilen Einmalartikels zum Verschließen der Reinigungsöffnung bzw. der Reinigungsöffnungen auf das Absaugrohr aufgeschoben wird. Wird das Verschlussrohr jedoch mehrfach verwendet, muss auch das Verschlussrohr gereinigt und sterilisiert werden. Da das Verschlussrohr ggf. nicht wesentlich kürzer als das Absaugrohr ist, stellen sich hierbei bezüglich der Reinigung und Desinfektion grundsätzlich dieselben Probleme wie bei dem Absaugrohr. Daher weist auch das Verschlussrohr bevorzugt eine oder mehrere Reinigungsöffnungen auf, durch die die mechanische Reinigung und die Dampfsterilisation vereinfacht und verbessert werden. Auch die Verschlussrohr-Reinigungsöffnung kann als Längsschlitz ausgebildet sein, beispielsweise als ein einziger Längsschlitz über die gesamte Länge des Verschlussrohres. Das Verschlussrohr ist in diesem Fall radial leicht federnd und kann mit einer geringen Vorspannung auf das Absaugrohr aufgesteckt werden, so dass es letzteres fest umklammert und die Reinigungsöffnung bzw. Öffnungen flüssigkeitsdicht verschließt.

Gemäß einer bevorzugten Ausgestaltung ist die Absaugöffnung bzw. sind die Absaugöffnungen in dem Verschlussrohr vorgesehen. Wenn das Verschlussrohr annähernd dieselbe axiale Länge aufweist wie das Absaugrohr, weisen sowohl das Absaugrohr als auch das Verschlussrohr an ihren distalen Enden jeweils Absaugöffnungen auf, die im Schließzustand des Verschlussrohres miteinander fluchten, also radial durchgehend geöffnet sind. Die Absaugöffnungen können auch ausschließlich an dem Verschlussrohr vorgesehen sein.

Vorzugsweise beträgt der Öffnungswinkel der Reinigungsöffnung in einer Radialebene mindestens 30°. Da Absaugrohre einen kleinen Außendurchmesser von wenigen Millimetern haben, muss der Öffnungswinkel des Reinigungsschlitzes ausreichend groß sein, um insbesondere für die mechanische Reinigung einen ausreichend weiten Zugang zum Absaugrohr-Inneren zu gewährleisten. Der Öffnungswinkel sollte möglichst groß sein, jedoch so klein, dass die Stabilität des Absaugrohres und die Abdichtung im Schließzustand nicht signifikant verschlechtert werden.

Vorzugsweise sind dem Absaugrohr und dem Verschlussrohr zusammenwirkende Befestigungselemente zugeordnet, durch die das Absaugrohr an dem Verschlussrohr im Schließzustand festgesetzt ist. Derartige Befestigungselemente können durch die mechanischen Bauteile eines Bajonetteverschlusses gebildet werden, können jedoch auch durch ein Außengewinde an dem Absaugrohr und ein Innengewinde an dem Verschlussrohr gebildet sein. An einem dem Absaugrohr zugeordneten Handgriff kann ein Innengewinde vorgesehen sein, das mit einem Außengewinde des Verschlussrohres zusammenwirkt. Als Befestigungselemente können auch einfache Führungen dienen, die lediglich eine Verdrehsicherung darstellen, jedoch nicht die eigentliche axiale Fixierung des Verschlussrohres gegenüber dem Absaugrohr vornehmen. Das Verschlussrohr kann annähernd die gleiche axiale Länge aufweisen, wie das Absaugrohr und an seinem distalen Ende verschlossen sein. Das Verschlussrohr umfasst dann das Absaugrohr vollständig.

Vorzugsweise ist die Reinigungsöffnung proximal und getrennt von der Absaugöffnung angeordnet. Im Bereich der Absaugöffnungen ist auch ohne Reinigungsöffnungen eine ausreichende mechanische Reinigung und Sterilisierbarkeit sichergestellt.

In bevorzugter Ausgestaltung weist das Verschlussrohr ein transparentes Fenster auf oder ist vollständig transparent ausgebildet. Der behandelnde Arzt kann durch das Verschlussrohr und durch die Reinigungsöffnung des Absaugrohres das Innere des Absaugrohres einsehen und auf diese Weise den Absaug-Vorgang beobachten.

Im folgenden werden unter Bezugnahme auf die Zeichnungen zwei Ausführungsbeispiele der Erfindung näher erläutert.

Es zeigen:
- Fig. 1: Einen Längsschnitt einer Absaugkanüle in Form einer Liposuktions-Kanüle mit einem Absaugrohr und einem Verschlussrohr,
- Fig. 2: einen ersten Querschnitt entlang der Linie II II der Absaugkanüle der Fig. 1,
- Fig. 3: einen Querschnitt in der Ebene III III der Absaugkanüle der Fig. 1,
- Fig. 4: ein zweites Ausführungsbeispiel einer Absaugkanüle im Längsschnitt, und
- Fig. 5: ein drittes Ausführungsbeispiel einer Absaugkanüle im Längsschnitt.

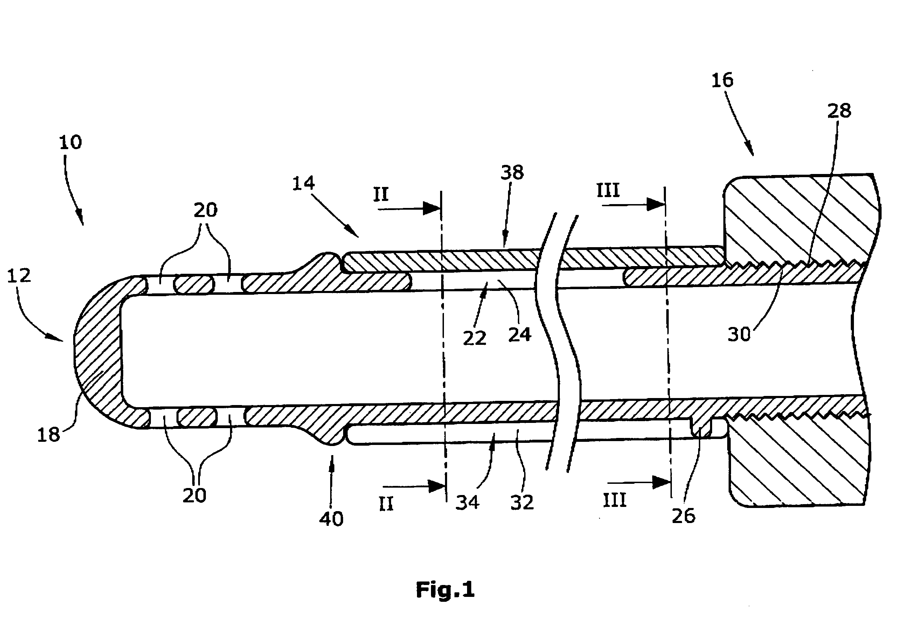

In den Figuren 1 - 3 ist eine Absaugkanüle 10 in Form einer Liposuktionskanüle zum Absaugen von Körperfett dargestellt. Die Absaugkanüle 10 besteht im wesentlichen aus einem Absaugrohr 12, einem als Reinigungsöffnungs-Verschluss dienenden Verschlussrohr 14 und einem von außen auf das Absaugrohr 12 proximal aufschraubbaren Handgriff 16. Das Absaugrohr 12, das Verschlussrohr 14 und der Handgriff 16 sind aus nicht rostendem Edelstahl gefertigt.

Das im Querschnitt kreisrunde Absaugrohr 12 weist an seinem distalen Ende eine abgerundete Kappe 18 auf. Am distalen Ende des Absaugrohres sind im Bereich des Rohrmantels mehrere Absaugöffnungen 20 vorgesehen. Es kann auch nur eine einzige Absaugöffnung vorgesehen sein, die auch am axialen Ende des Absaugrohres 12 angeordnet sein kann.

Das Absaugrohr 12 weist in seinem mittleren Bereich als Reinigungsöffnung 22 einen Längsschlitz 24 auf, der sich durchgehend nahezu bis zu dem Handgriff 16 erstreckt. Es können auch mehrere Reinigungsöffnungen in Form mehrerer kürzerer Längsschlitze vorgesehen sein, die axial aneinander gereiht sind und durch Materialstege des Absaugrohres 12 voneinander getrennt sind. Hierdurch würde eine größere Stabilität des Absaugrohres in Umfangsrichtung gegen radiale Belastungen erzielt.

An seinem proximalen Ende weist das Absaugrohr 12 einen radial nach außen weisenden Sicherungssteg 26 auf, der der Verdrehsicherung des Verschlussrohres 14 gegenüber dem Absaugrohr 12 dient. Proximal an den Sicherungssteg 26 schließt sich ein Außengewinde 28 an, das mit einem entsprechenden Innengewinde 30 des Handgriffes 16 zusammenwirkt. Der Sicherungssteg 26, das Absaugrohr-Außengewinde 28 und das Handgriff-Innengewinde 30 bilden zusammen Befestigungselemente, die das Absaugrohr 12 und das Verschlussrohr 14 gegeneinander bezüglich aller Freiheitsgrade festsetzen.

Das Verschlussrohr 14 ist im Querschnitt ebenfalls kreisrund und entspricht mit seinem Innendurchmesser ungefähr dem Außendurchmesser des Absaugrohres 12. Das Verschlussrohr 14 weist einen durchgehenden axialen Längsschlitz 32 auf, der eine Reinigungsöffnung 34 bildet. Sowohl der Absaugrohr-Längsschlitz als auch der Verschlussrohr-Längsschlitz 32 weisen einen Öffnungswinkel von ungefähr 70° auf. Das geschlossene Segment des Verschlussrohres 14 bildet einen Reinigungsöffnungs-Verschluss 38, der in den Figuren 1 - 3 im Schließzustand dargestellt ist, da er die Reinigungsöffnung 22 des Absaugrohres 12 vollständig und flüssigkeitsdicht verschließt. Am proximalen Ende des Verschlussrohres 14 weist das Absaugrohr 12 einen wulstartigen, im Längsschnitt rampenförmigen Anschlagring 40 auf, der einen axialen Anschlag für das Verschlussrohr 14 an dem Absaugrohr 12 bildet. Der Anschlagring 40 ist rampenförmig und an allen Kanten abgerundet ausgebildet, um Gewebe-Traumatisierungen bei der Liposuktion zu vermeiden. Dies gilt für alle Kanten, die bei einer Liposuktion mit Gewebe in Kontakt kommen können. Falls es gewünscht es, kann der Absaugrohr-Längsschlitz 24 auch bis zum distalen Ende des Absaugrohres 12 gezogen sein. In diesem Fall müssen jedenfalls im Bereich des Absaugrohr-Längsschlitzes in dem Verschlussrohr 14 entsprechende Absaugöffnungen vorhanden sein, wenn in diesem Bereich eine Absaugung gewünscht ist.

Zur Säuberung und Desinfektion wird die Absaugkanüle 10 demontiert. Dies erfolgt dadurch, dass zunächst der Handgriff 16 von dem Absaugrohr 12 abgeschraubt wird. Anschließend wird das Verschlussrohr 14 in proximal-axialer Richtung von dem Absaugrohr 12 abgezogen. Das Absaugrohr 12 kann nun mechanisch problemlos durch die Reinigungsöffnung 22 hindurch gereinigt werden, beispielsweise durch eine entsprechende Bürste und unter zu Hilfenahme einer Reinigungsflüssigkeit, die beide durch die Reinigungsöffnung 22 eingeführt werden können.

Das gleiche gilt für die Reinigung des Verschlussrohres 14, das durch seine Reinigungsöffnung 34 hindurch beispielsweise mit einer Bürste und einer Reinigungsflüssigkeit gründlich mechanisch gereinigt werden kann. Um eine gute Zugänglichkeit für alle Bereiche für die Reinigung sicherzustellen, und um zu vermeiden, dass sich in kleinen Nischen Rückstände festsetzen können, die bei der mechanischen Reinigung nicht oder nur schlecht erfasst werden können, sind alle Bereiche, die gereinigt werden müssen, ebenfalls großzügig abgerundet ausgebildet, d. h. mit einem möglichst großen Radius versehen.

Nach der mechanischen Reinigung erfolgt die Dampf-Sterilisation des Absaugrohres 12, des Verschlussrohres 14 und des Handgriffes 16 in demoniertem Zustand. Erst nach der Dampf-Sterilisation werden die drei genannten Teile wieder zu einer betriebsbereiten Absaugkanüle 10 zusammengesetzt. Hierbei wird zunächst das Verschlussrohr 14 auf das Absaugrohr 12 aufgeschoben, und der Handgriff 16 anschließend auf das Absaugrohr 12 aufgeschraubt. Hierdurch wird das Verschlussrohr 14 axial auf dem Absaugrohr 12 fixiert, während die Fixierung in Umfangsrichtung durch den Sicherungssteg 26 erfolgt.

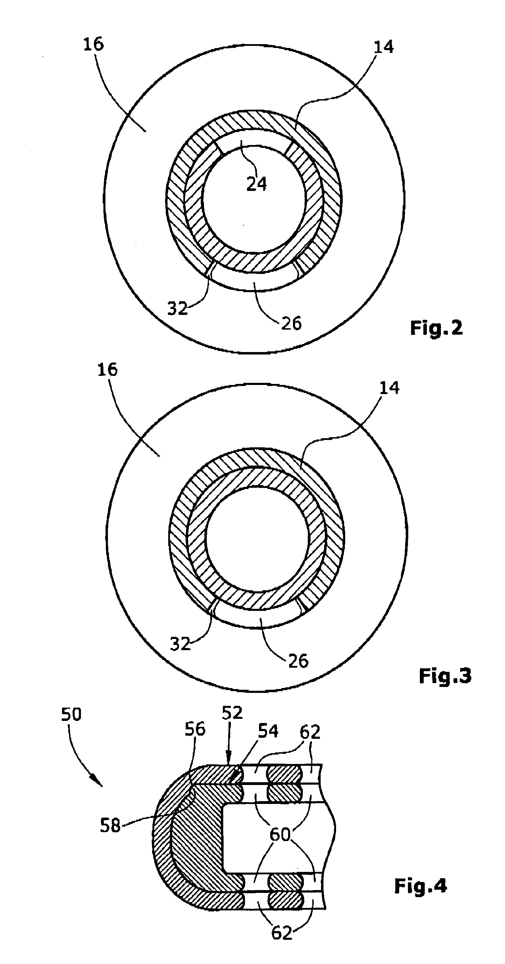

In der Fig. 4 ist ein zweites Ausführungsbeispiel einer Absaugkanüle 50 dargestellt, bei der das Verschlussrohr 52 ähnlich ausgebildet ist wie das Absaugrohr 54, nämlich an seinem distalen Ende jeweils vollständig durch eine Kappe verschlossen.

Das Absaugrohr 54 weist Absaugöffnungen 60 auf, die mit entsprechenden Absaugöffnungen 62 des Verschlussrohres 52 fluchten.

Eine Verdrehsicherung wird durch eine absaugrohrseitige Nase 56 am distalen Ende des Absaugrohres 54 gebildet, die in eine entsprechende innenseitige Ausnehmung 58 des Verschlussrohres 52 eingreift. Proximal der Absaugöffnungen 60, 62 ist die Absaugkanüle 50 ebenso aufgebaut, wie die des in den Figuren 1 - 3 dargestellten Ausführungsbeispieles einer Absaugkanüle 10.

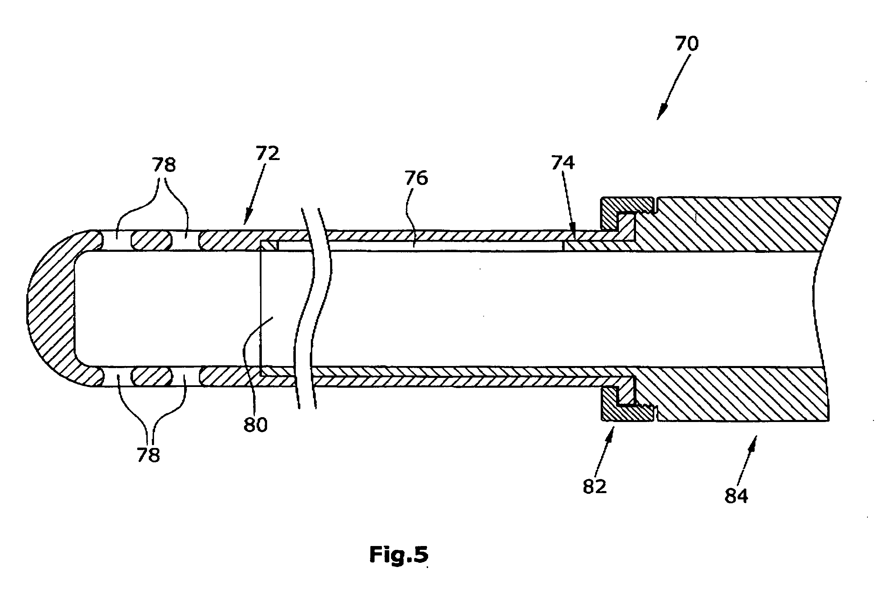

In der Figur 5 ist ein drittes Ausführungsbeispiel einer Absaugkanüle 70 dargestellt, bei der das Verschlussrohr 72 als Einmalartikel ausgebildet und aus transparentem Kunststoff gefertigt ist. Grundsätzlich kann das Kunststoff-Verschlussrohr 72 aber auch mehrfach verwendbar ausgelegt werden. Das Absaugrohr 74 besteht aus Stahl und ist mehrfach verwendbar. Das Absaugrohr 74 weist daher eine als Längsschlitz ausgebildete Reinigungsöffnung 76 auf. Im vorliegenden Beispiel sind radiale Absaugöffnungen 78 am distalen geschlossenen Ende des Verschlussrohres 72 vorgesehen. Das Absaugrohr 74 weist an seinem distalen Ende eine axiale Öffnung 80 auf, durch die das biologische Gewebe abgesaugt wird. Das Absaugrohr 74 dient darüber hinaus der mechanischen Stabilisierung der Absaugkanüle 70 über annähernd seine gesamte Länge. Auf diese Weise wird eine starke Nachgiebigkeit der Absaugkanüle 70 im Bezug auf Biegemomente vermieden, die ein Kunststoff-Verschlussrohr 72 alleine über eine gewisse Länge nicht gewähren kann.

Das Einmalartikel-Verschlussrohr 72 wird durch eine Stahl-Überwurfmutter 82 an dem Griff 84 des Absaugrohres 74 fixiert. Die Fixierung des Einmalartikel-Verschlussrohres an dem Absaugrohr kann auf vielfältige Weise erfolgen, beispielsweise durch Rastelemente an dem Verschlussrohr und dem Absaugrohr, durch separate Klemmhebel etc.

Das Verschlussrohr 72 wird nur einmal verwendet und danach nicht mehr sterilisiert, sondern weggeworfen. Das Verschlussrohr 72 ist preiswert im Spritzguss herstellbar. Durch die Ausbildung des Verschlussrohres als Einmalartikel kann das Verschlussrohr ausschließlich im Hinblick auf seine Suktions-Funktionen konstruiert werden. Eine gute Zugänglichkeit für eine mechanische Reinigung und Sterilisation ist bei der Konstruktion des Verschlussrohres nicht zu berücksichtigen. Insbesondere entfällt die Notwendigkeit, auch an dem Verschlussrohr 72 Reinigungsöffnungen vorzusehen.

Selbstverständlich können die Reinigungsöffnungen auch ausschließlich in dem Absaugrohr vorgesehen sein, ähnlich dem Absaugrohr 12 der Figur 1, während das als Einmalartikel ausgebildete Verschlussrohr ähnlich dem in der Figur 1 dargestellten Verschlussrohr 14 ausgebildet ist, jedoch keine eigene Reinigungsöffnung aufweist.

## Patentansprüche

1. Absaugkanüle (10) zum Absaugen von biologischem Gewebe aus einem menschlichen Körper, mit
einem Absaugrohr (12) und mindestens einer Absaugöffnung (20),
wobei das Absaugrohr (12) mindestens eine Reinigungsöffnung (22) und einen Reinigungsöffnungs-Verschluss (38) aufweist, der in seinem Schließzustand die Reinigungsöffnung (22) verschließt und in seinem Öffnungszustand die Reinigungsöffnung (22) freigibt,
**dadurch gekennzeichnet,**
**dass** der Reinigungsöffnungs-Verschluss (38) von einem Verschlussrohr (14) gebildet wird, das im Schließzustand außen auf dem Absaugrohr (12) sitzt und die Reinigungsöffnung (22) abdeckt.

2. Absaugkanüle (70) nach Anspruch 1, **dadurch gekennzeichnet, dass** das Verschlussrohr (72) ein Einmalartikel ist.

3. Absaugkanüle (70) nach Anspruch 2, **dadurch gekennzeichnet, dass** das Absaugrohr (74) aus Metall besteht und das Verschlussrohr (72) aus Kunststoff besteht.

4. Absaugkanüle (10) nach einem der Ansprüche 1-3, **dadurch gekennzeichnet, dass** die Reinigungsöffnung (22) von einem Längsschlitz (24) in dem Absaugrohr (12) gebildet wird.

5. Absaugkanüle (10) nach einem der Ansprüche 1-4, **dadurch gekennzeichnet, dass** das Verschlussrohr (14) mindestens eine Reinigungsöffnung (34) aufweist.

6. Absaugkanüle (10) nach einem der Ansprüche 1-5, **dadurch gekennzeichnet, dass** die Absaugöffnung in dem Verschlussrohr (14) vorgesehen ist.

7. Absaugkanüle (10) nach einem der Ansprüche 1-6, **dadurch gekennzeichnet, dass** dem Absaugrohr (12) und dem Verschlussrohr (14) zusammenwirkende Befestigungselemente zugeordnet sind.

8. Absaugkanüle (10) nach einem der Ansprüche 1-7, **dadurch gekennzeichnet, dass** die Reinigungsöffnung (22) proximal der Absaugöffnung (20) angeordnet ist.

9. Absaugkanüle (10) nach einem der Ansprüche 1-8, **dadurch gekennzeichnet, dass** das Verschlussrohr (72) ein transparentes Fenster aufweist oder transparent ist.

## Claims

1. Suction canula (10) for suctioning biological tissue from a human body, comprising:
a suction tube (12) and at least one suction opening (20),
said suction tube (12) comprising at least one cleaning opening (22) and a cleaning opening closure (38) which, in its closed state, closes the cleaning opening (22) and which, in its open state, clears the cleaning opening (22),
**characterized in that**
the cleaning opening closure (38) is formed by a closing tube (14) which, in the closed state, sits on the outside of the suction tube (12) and covers the cleaning opening (22).

2. Suction canula (70) of claim 1, **characterized in that** the closing tube (72) is a disposable article.

3. Suction canula (70) of claim 2, **characterized in that** the suction tube (74) is made of metal and the closing tube (72) is made of plastics.

4. Suction canula (10) of one of claims 1-3, **characterized in that** the cleaning opening (22) is formed by a longitudinal slot (25) in the suction tube (12).

5. Suction canula (10) of one of claims 1-4, **characterized in that** closing tube (14) comprises at least one cleaning opening (34).

6. Suction canula (10) of one of claims 1-5, **characterized in that** the suction opening is provided in the closing tube (14).

7. Suction canula (10) of one of claims 1-6, **characterized in that** cooperating fastening elements are associated with the suction tube (12) and the closing tube (14).

8. Suction canula (10) of one of claims 1-7, **characterized in that** the cleaning opening (22) is provided proximal of the suction opening (20).

9. Suction canula (10) of one of claims 1-8, **characterized in that** the closing tube (72) has a transparent window or is transparent.

## Revendications

1. Canule d'aspiration (10) pour l'aspiration de tissu biologique d'un corps humain, comprenant
un tube d'aspiration (12) et au moins une ouverture d'aspiration (20),
ledit tube d'aspiration (12) comprenant au moins une ouverture de nettoyage (22) et une fermeture (38) de l'ouverture de nettoyage, qui, dans son état fermé ferme ladite ouverture de nettoyage (22) et débloque ladite ouverture de nettoyage (22) dans son état ouvert,
**caractérisée en ce que**
la fermeture (38) de l'ouverture de nettoyage est formée par un tube de fermeture (14) qui est placé sur l'extérieur du tube d'aspiration (12) et couvre l'ouverture de nettoyage (22).

2. Canule d'aspiration (70) selon la revendication 1, **caractérisée en ce que** le tube de fermeture (72) est un article jetable.

3. Canule d'aspiration (70) selon la revendication 2, **caractérisée en ce que** le tube d'aspiration (74) est en métal et le tube de fermeture (72) est en matière plastique.

4. Canule d'aspiration (10) selon l'une des revendications 1 - 3, **caractérisée en ce que** l'ouverture de nettoyage (22) est formée par une fente longitudinale (24) dans le tube d'aspiration (12).

5. Canule d'aspiration (10) selon l'une des revendications 1 - 4, **caractérisée en ce que** le tube de fermeture (14) comprend au moins une ouverture de nettoyage (34).

6. Canule d'aspiration (10) selon l'une des revendications 1 - 5, **caractérisée en ce que** l'ouverture d'aspiration est prévue dans le tube de fermeture (14).

7. Canule d'aspiration (10) selon l'une des revendications 1 - 6, **caractérisée en ce que** des éléments de fixation coopérant sont attribués au tube d'aspiration (12) et au tube de fermeture (14).

8. Canule d'aspiration (10) selon l'une des revendications 1 - 7, **caractérisée en ce que** l'ouverture de nettoyage (22) est prévue proximal de l'ouverture d'aspiration (20).

9. Canule d'aspiration (10) selon l'une des revendications 1 - 8, **caractérisée en ce que** le tube de fermeture (72) comprend une fenêtre transparente ou qu'il est transparent.
